# EUROPÄISCHE PATENTSCHRIFT

(11) **EP 3 122 718 B1**
(45) Veröffentlichungstag und Bekanntmachung des Hinweises auf die Patenterteilung: **28.02.2018**
(21) Anmeldenummer: 15711525.4
(22) Anmeldetag: 24.03.2015
(51) Int. Cl.: C07C 263/10, C07C 265/14

(54) **VERFAHREN ZUR HERSTELLUNG VON ISOCYANATEN**
METHOD FOR MANUFACTURING ISOCYANATES
PROCÉDÉ DESTINÉ À LA FABRICATION D'ISOCYANATES

(30) Priorität: 27.03.2014 EP 14162003
(43) Veröffentlichungstag der Anmeldung: 01.02.2017
(73) Patentinhaber: Covestro Deutschland AG, 51373 Leverkusen (DE)
(72) Erfinder: KNAUF, Thomas, 41542 Dormagen (DE); RAUSCH, Andreas Karl, 41564 Kaarst (DE); BJOERNDAHL, Charles, 24613 Aukrug-Böken (DE); EHLERS, Matthias, 25709 Marne (DE); PLATHEN, Peter, 47829 Krefeld (DE); ALVAREZ HERRERO, Carlos, 43007 Tarragona (ES); MUNOZ VELASCO, Francisco, 43480 Tarragona (ES)
(74) Vertreter: Levpat
(86) Internationale Anmeldenummer: PCT/EP2015/056183
(87) Internationale Veröffentlichungsnummer: WO 2015/144658

(56) Entgegenhaltungen:
- WO-A1-01/17951
- WO-A1-2013/029918

## Beschreibung

Die Erfindung betrifft ein Verfahren zur Herstellung von Isocyanaten durch Phosgenierung der korrespondierenden Amine, bei dem Probleme infolge der Bildung von Ablagerungen in Apparaten der Reaktionsstrecke während der Inbetriebnahme (dem Anfahren) und der Außerbetriebnahme (dem Abfahren) des Verfahrens durch verfahrenstechnische Maßnahmen, insbesondere der Sicherstellung eines Überschusses an Phosgen gegenüber dem zu phosgenierenden Amin während der kritischen An- und Abfahrschritte des Verfahrens, vermieden werden.

Die großtechnische Herstellung von Polyisocyanaten durch Umsetzung der korrespondierenden Amine mit Phosgen ist seit längerem aus dem Stand der Technik bekannt, wobei die Reaktion in der Gas- oder Flüssigphase sowie diskontinuierlich oder kontinuierlich durchgeführt werden (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise Ullmanns Encyklopädie der technischen Chemie, 4. Aufl. (1977), Band 13, S. 351 bis 353 sowie G. Wegener et. al. Applied Catalysis A: General 221 (2001), p. 303 - 335, Elsevier Science B.V. Es kommen dabei sowohl aromatische Isocyanate wie beispielsweise Methylendiphenyldiisocyanat (MMDI - "monomeres MDI"), Polymethylen-Polyphenylen-Polyisocyanat (ein Gemisch aus MMDI und höheren Homologen, PMDI, "polymeres MDI") oder Toluylendiisocyanat (TDI) als auch aliphatische Isocyanate wie z. B. Hexamethylendiisocyanat (HDI) oder Isophorondiisocyanat (IPDI) weltweit zum Einsatz.

Die moderne großtechnische Herstellung von Polyisocyanaten erfolgt kontinuierlich, und die Reaktionsführung erfolgt als adiabate Phosgenierung wie in EP 1 616 857 B2 beschrieben. Unerwünschte Ablagerungen und Nebenprodukte im Reaktor werden durch richtige Wahl von Reaktionstemperatur und Druck vermieden. Im Mischraum ist ein molarer Überschuss von Phosgen zu den primären Aminogruppen zu gewährleisten. Eine dreistufige Phosgenierstraße ist in EP 1 873 142 B1 beschrieben, bei der der Druck zwischen der ersten Stufe eines dynamischen Mischers und der zweiten Stufe eines ersten Phosgenierreaktors gleich bleibt oder ansteigt und in der dritten Stufe in einem Apparat zur Phosgenabtrennung der Druck niedriger ist als in der zweiten Stufe.

WO 2013/029918 A1 beschreibt ein Verfahren zur Herstellung von Isocyanaten durch Umsetzung der korrespondierenden Amine mit Phosgen, das auch bei unterschiedlichen Auslastungen der Anlage ohne Probleme durchgeführt werden kann, insbesondere soll auch bei Fahren der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Das erfindungsgemäße Verfahren soll es ermöglichen, eine bestehende Anlage bei unterschiedlichen Auslastungen mit gleichbleibender Produkt- und Verfahrensqualität zu betreiben. Dies soll die Anschaffung mehrerer Anlagen mit unterschiedlichen Nennkapazitäten einsparen.

Die Anmeldung lehrt, dass wesentliche Parameter einer Phosgenierung, wie insbesondere die Verweilzeiten der Reaktionspartner in den einzelnen Apparaten, für den Betrieb der Produktionsanlage bei Nennkapazität optimiert sind, was zu Problemen hinsichtlich Ausbeute und Produktreinheit führen kann, wenn die Anlage bei geringerer als Nennkapazität betrieben wird (vgl. S. 2, Z. 20 bis 36). Um die optimierten - engen - Verweilzeitfenster auch bei Teilauslastung (d. h. gegenüber Betrieb bei Nennkapazität verringertem Aminstrom) erreichen zu können, wird vorgeschlagen, entweder den Phosgenstrom und/oder den Inertenanteil zu erhöhen (vgl. S. 3, Z. 5 bis 19), und zwar bevorzugt so, dass der Gesamtmengenstrom aller Komponenten im Wesentlichen dem bei Nennkapazität entspricht (vgl. S. 6, Z. 4 bis 8). Die Anmeldung erwähnt zwar An- und Abfahrprozesse in der Beschreibung des Hintergrunds der beanspruchten Erfindung auf S. 2, offenbart weder eine technische Lehre zum konkreten Handeln, wie denn eine nicht in Betrieb befindliche Produktionsanlage (d. h. Aminstrom und Phosgenstrom sind gleich null) am vorteilhaftesten auf den angestrebten Betriebszustand der Nennkapazität gebracht wird, noch eine technische Lehre zum konkreten Handeln, wie denn eine in Betrieb befindliche Produktionsanlage am vorteilhaftesten außer Betrieb (d. h. Aminstrom und Phosgenstrom sind gleich null) genommen wird. Die in der Anmeldung offenbarten technischen Maßnahmen (d. h. die Erhöhung des Phosgenstroms und/oder des Inertenanteils) sind ausschließlich im Zusammenhang mit der Problematik *des Betriebs* (d. h. der Aminstrom ist signifikant größer als null) einer Produktionsanlage bei geringerer als Nennkapazität bzw. mit der Problematik, wie eine bei Nennkapazität betriebene Anlage vorteilhafterweise auf den Betrieb bei geringerer als Nennkapazität umgestellt werden kann (siehe die Beispiele), zu sehen. Auf die Reihenfolge der Inbetriebnahme einzelner Ströme beim Anfahren bzw. der Außerbetriebnahme einzelner Ströme beim Abfahren geht die Schrift nicht ein.

Der Reaktionsaustrag aus der Phosgenierstraße kann, wie in EP 1 546 091 B1 beschrieben, aufgearbeitet werden. Die Aufarbeitung des Reaktionsproduktes erfolgt in einem Schichtverdampfer, bevorzugt einem Fallfilmverdampfer, in dem Phosgen und HCl schonend verdampft werden.

US 5 136 087 (B) beschreibt ebenso die Entfernung von Phosgen aus der Reaktionsmischung der Phosgenierung mittels eines inerten Lösungsmitteldampfes, der aus der Lösungsmittelrückgewinnung der Phosgenieranlage stammen kann.

Eine mögliche Ausführungsform der Lösungsmittelabtrennung und -rückgewinnung ist in EP 1 854 783 A2 beschrieben. Di- und Polyisocyanate der Diphenylmethanreihe (MDI), die durch Umsetzung von in einem Lösungsmittel gelösten entsprechenden Aminen mit Phosgen gewonnen wurden, werden zuerst von Chlorwasserstoff und überschüssigem Phosgen befreit, und anschließend wird eine destillative Auftrennung dieser Rohlösung in Isocyanate und Lösungsmittel durchgeführt. Das Lösungsmittel wird in den Prozess zurückgeführt zur Herstellung von Lösungen der Einsatzstoffe der Polyisocyanatherstellung. In Fall der Herstellung von MDI unter Verwendung von Monochlorbenzol als Lösungsmittel kann diese destillative Auftrennung vorteilhaft so erfolgen, dass die Isocyanat-Rohlösung in zwei Schritten in ein Sumpfprodukt enthaltend mindestens 95 Gew.-% an Isocyanat, bezogen auf das Gewicht des Isocyanat enthaltenden Stroms, aufgearbeitet wird, das anschließend bevorzugt in weiteren Schritten von Leichtsiedern befreit wird. Im ersten Schritt werden dabei bevorzugt durch eine Flashdestillation bei absoluten Drücken von 600 - 1200 mbar und Sumpftemperaturen von 110 °C - 170 °C 60 - 90 % des in der Isocyanat-Rohlösung enthaltenen Lösungsmittels abgetrennt, wobei die Brüden in einer Destillationskolonne mit 5 - 20 Trennstufen und 10 - 30 % Rücklauf aufgearbeitet werden, so dass ein Lösungsmittel enthaltender Strom mit einem Diisocyanatgehalt von < 100 ppm, bevorzugt < 50 ppm, besonders bevorzugt < 20 ppm, bezogen auf das Gewicht des Lösungsmittel enthaltenden Stroms, erzielt wird. Im zweiten Schritt wird das restliche Lösungsmittel bis auf einen Restgehalt von 1 - 3 Gew.-% im Sumpfprodukt bei Drücken von 60 - 140 mbar absolut und Sumpftemperaturen von 130 °C - 190 °C abgetrennt. Die Brüden können ebenfalls in einer Destillationskolonne mit 5 - 20 Trennstufen und 10 - 40 % Rücklauf aufgearbeitet werden, so dass ein Lösungsmittel enthaltender Strom mit einem Diisocyanatgehalt von < 100 ppm, bevorzugt < 50 ppm, besonders bevorzugt < 20 ppm, bezogen auf das Gewicht des Lösungsmittel enthaltenden Stroms, erzielt wird oder nach Kondensation wieder als Zulauf in den ersten Destillationsschritt rückgeführt werden. In gleicher Weise können die in den folgenden Schritten abgetrennten Destillatströme wieder als Zulauf in den ersten Destillationsschritt rückgeführt werden.

Bei geeigneter Auslegung der Destillation weist das zurückgewonnene Lösungsmittel die vorab genannten Gehalte an Diisocyanat auf. Zusätzlich kann durch Verwendung geeigneter technischer Maßnahmen die Lösungsmittelqualität bzgl. Diisocyanatgehalt weiter gesteigert werden, indem z. B. Diisocyanat enthaltende Lösungsmittelnebel oder -tröpfchen in den Brüden der ein oder mehrstufigen destillativen Lösungsmittelabtrennung mittels Demister, Prallblech, Hydrozyklon oder durch Quenchen (Abspritzen) mit frischem oder rezykliertem Lösungsmittel ganz oder teilweise abgetrennt werden. Kombinationen der vorgenannten Maßnahmen sind auch möglich.
In EP 1 854 783 A2 ist beschrieben, welche Qualitätsanforderungen für ein Lösungsmittel für ein Verfahren zur Herstellung von Polyisocyanaten bestehen. Es wurde gefunden, dass die Reinheit des im Kreislauf geführten Lösungsmittels, das zur Herstellung der in der Phosgenierung eingesetzten Aminlösung verwendet wird, für die Nebenproduktbildung im Roh-Isocyanat von entscheidender Bedeutung ist. Selbst ein Gehalt von nur 100 ppm Phosgen oder 100 ppm Diisocyanat, bezogen auf das Gewicht des Lösungsmittels, führt zu nachweisbarer Nebenproduktbildung im Roh-Isocyanat. Während dies bei destillierten Isocyanaten, d. h., bei den als Kopfprodukt gewonnen Isocyanaten, zu einer Ausbeuteverminderung führt, wird bei den als Sumpfprodukt gewonnenen Isocyanaten, wie z. B. den Di- und Polyisocyanaten der Diphenylmethanreihe, dadurch eine unerwünschte Beeinflussung der Qualität (Farbe) und des Reaktionsverhaltens bewirkt. Dies ist beispielsweise durch chlorierte Nebenkomponenten sowie erhöhten Eisengehalt nachweisbar.

Tetrachlorkohlenstoff als Verunreinigung des Lösungsmittels gelangt über das Phosgen in den Phosgenierungskreislauf und reichert sich im Lösungsmittel über den Lösungsmittelkreislauf an. Mit der Zeit pendelt sich eine gleichbleibende Konzentration von Tetrachlorkohlenstoff ein, die von den Verlusten von Tetrachlorkohlenstoff über den Austrag mit dem Abgas geprägt ist. Je nach Prozessbedingungen hat ein in der Phosgenierung eingesetztes Lösungsmittel, das nicht frisch zugeführt wurde, sondern aus prozessinternen Rezyklierungsströmen stammt, einen Massengehalt an Tetrachlorkohlenstoff von 0,01 % bis 5 %. unter Umständen sogar bis zu 20 %, bezogen auf die Gesamtmasse des Lösungsmittels inklusive aller Verunreinigungen.

DE-A-19942299 beschreibt ein Verfahren zur Herstellung von Mono- und Oligoisocyanaten durch Phosgenierung der entsprechenden Amine, wobei eine katalytische Menge eines Monoisocyanats in einem inerten Lösungsmittel mit Phosgen vorgelegt wird, das Amin, normalerweise in Lösungsmittel gelöst, zugesetzt wird und das erhaltene Reaktionsgemisch mit Phosgen umgesetzt wird. Die intermediäre Bildung von schwerlöslichen Suspensionen wird vermieden. Das gewünschte Isocyanat wird dabei bei vollständigem Umsatz des Amins in hohen Ausbeuten und hoher Selektivität in deutlich verkürzten Reaktionszeiten gebildet, ohne dass symmetrisch substituierter N,N'-Harnstoff aus dem Amin als Nebenprodukt gebildet wird. Das Verfahren ist allerdings, vor allem durch Einsatz des zusätzlichen Monoisocyanates, das später wieder abgetrennt werden muss, vergleichsweise kompliziert und energieaufwändig.

Von wenigen Ausnahmen abgesehen befasst sich der beschriebene Stand der Technik nur mit Verfahren, die sich im Normalbetrieb befinden. Anfahrprozesse bis zum Erreichen eines stabilen Betriebszustandes bei dem angestrebten Soll-Mengenstrom des Amins (sogenannte "Anfahrzeit") oder Abfahrprozesse bis zum Erreichen der kompletten Abschaltung (sogenannte "Abfahrzeit") werden in den Schriften zu kontinuierlichen großtechnischen Verfahren nicht berücksichtigt. Lediglich in Schriften, in denen die diskontinuierliche Phosgenierung beschrieben wird, werden die Anfahrphasen genauer berücksichtigt; siehe bspw. US 2,908,703 und US 2,822,373. Auch unerwartete Betriebsstillstände (z. B. ein plötzlich erzwungenes Abfahren der Anlage) führen kurzzeitig zu Verfahrensführungen, die wesentlich von denen im Normalbetrieb abweichen können.

Die vorliegende Erfindung befasst sich speziell mit solchen Abweichungen vom Normalbetrieb bei kontinuierlichen Verfahren zur Herstellung von Di- und Polyisocyanaten durch Phosgenierung der entsprechenden primären Amine in der Flüssigphase. An- und Abfahrzeiten treten im industriellen Alltag häufig auf und sind nicht zwangsläufig mit einem Öffnen oder einem anderen mechanischen Eingriff in die Phosgenieranlage verbunden, sondern nur mit dem Abstellen und erneuten Starten der Phosgenieranlage. Diese An- und Abfahrzeiten zeichnen sich in der Praxis dadurch aus, dass es zu Abweichungen im Verhältnis von Phosgen zu Amin kommen kann. Dies ist insbesondere dann zu beobachten, wenn die Menge an pro Zeiteinheit umzusetzenden Amin (der Amin-Mengenstrom) sehr klein ist im Vergleich zum Betrieb der Anlage bei Nennkapazität. Diese Schwankungen im Verhältnis von Phosgen zu Amin sind nachteilig, da sich vermehrt Feststoffe wie Polyharnstoff oder Aminhydrochlorid bilden und ausfallen können. Gleiches gilt für ungeplante, plötzliche Abschaltungen einer sich im Normalbetrieb befindlichen Phosgenieranlage.

Abweichungen vom Normalbetrieb, seien sie nun geplant oder das Resultat unerwarteter Vorfälle, können daher ein erhöhtes Risiko im Hinblick auf den reibungslosen Betrieb nach Wiederherstellung des Normalzustandes zur Folge haben; beispielsweise infolge vermehrter Bildung von Ablagerungen in Apparaten. Es bestand daher ein Bedarf nach einem Verfahren zur Herstellung von Isocyanaten, bei dem dieses Risiko durch geeignete Vorkehrungen minimiert wird.

Diesem Bedarf Rechnung tragend ist ein Gegenstand der vorliegenden Erfindung ein kontinuierliches Verfahren zur Herstellung eines Isocyanats (1) durch Umsetzung des korrespondierenden Amins **(2)** mit Phosgen **(3)** in einem inerten Lösungsmittel **(4)** in einer Reaktionsstrecke **(1000),** welche
(a) eine Mischzone **(1100)** zur Vermischung von Amin **(2),** Phosgen **(3)** und inertem Lösungsmittel **(4)**
   und
(b) eine strömungstechnisch nach der Mischzone **(1100)** angeordnete Reaktionszone **(1200),**
umfasst;
bei einer Soll-Temperatur T_{Soll}, wobei nacheinander die Schritte
(A) Anfahren der kontinuierlichen Produktion,
(B) kontinuierliche Produktion und
(C) Abfahren der kontinuierlichen Produktion, und bevorzugt
(D) Verdrängung des Phosgens **(3)** aus der Reaktionsstrecke **(1000)**
durchlaufen werden, und wobei
in Schritt (A)
(I) die Mischzone **(1100)** und die Reaktionszone **(1200)** zunächst
   (i) nur mit inertem Lösungsmittel **(4)** zumindest teilweise beschickt, dann auf T_{Soll} aufgeheizt und danach zusätzlich mit Phosgen **(3)** aber nicht mit Amin (2), bevorzugt zusammen mit weiterem inerten Lösungsmittel **(4),** beschickt werden;
      oder
   (ii) mit inertem Lösungsmittel **(4)** und Phosgen **(3)** ohne das Amin **(2)** zumindest teilweise beschickt und danach auf T_{Soll} aufgeheizt werden;
(II) erst im Anschluss an Schritt (A) (I) der Reaktionszone **(1200)** das Amin **(2)** sowie weiteres Phosgen **(3)** und weiteres inertes Lösungsmittel **(4)** über die Mischzone **(1100)** kontinuierlich zugeführt werden;
und in Schritt (C) zum Abfahren der kontinuierlichen Produktion zunächst nur die Zufuhr des Amins **(2)** beendet wird, während Phosgen **(3)** und inertes Lösungsmittel **(4)** noch weiter kontinuierlich zugeführt werden.

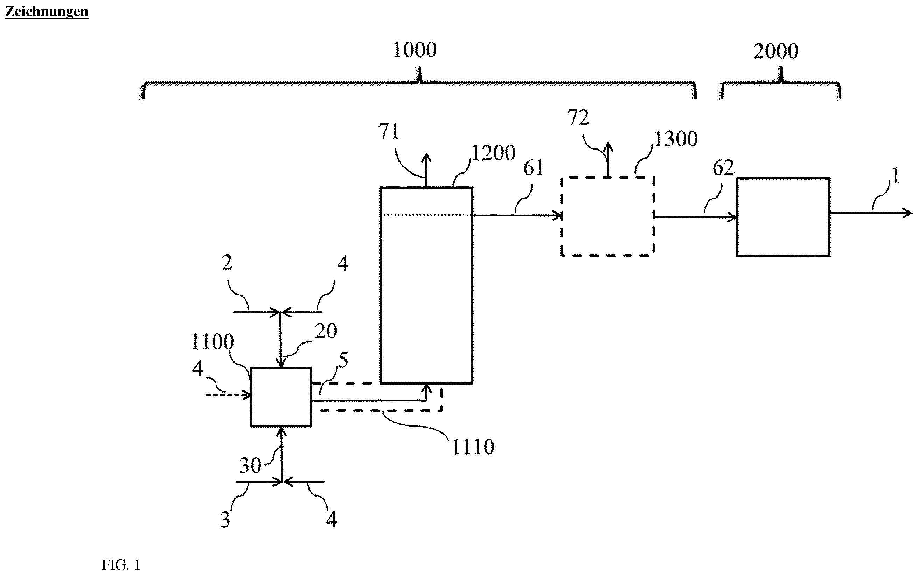

Die *Reaktionsstrecke **(1000)*** bezeichnet den Teil einer Phosgenierungsanlage, in dem die eigentliche Umsetzung von Amin mit Phosgen zum Isocyanat stattfindet, also den Reaktionsteil im Unterschied zum Aufarbeitungsteil **(2000).** Eine mögliche Ausgestaltung einer Reaktionsstrecke **1000** (mit daran anschließendem Aufarbeitungsteil **2000**) im Sinne der vorliegenden Erfindung zeigt FIG. 1. Optionale Apparate sind darin gestrichelt gezeichnet. Der Reaktionsteil umfasst erfindungsgemäß mindestens eine Mischzone **(1100)** und mindestens eine strömungstechnisch nach der Mischzone **(1100)** angeordnete Reaktionszone **(1200).** *Strömungstechnisch nach der Mischzone angeordnet* bedeutet, dass der Austrag **(5)** der Mischzone in die Reaktionszone **(1200)** fließt, wobei ggf. noch eine Verweilzeiteinrichtung **(1110)** zwischengeschaltet sein kann. Im erfindungsgemäßen Verfahren befindet sich während der Durchführung von Schritt (A) (I) kein Isocyanat in der Reaktionsstrecke **(1000).** Erst mit Beginn der Aminzufuhr in Schritt (A) (II) *bildet* sich erstmalig Isocyanat **(1).** Nach beendetem Schritt (C) ist die Reaktionsstrecke **(1000)** wieder frei von Isocyanat **(1),** sodass die Reaktionsstrecke **(1000)** während des Schrittes (A) (I) kein Isocyanat **(1)** aus dem vorigen Produktionszyklus enthält. Da auch kein Isocyanat **(1)** in der Reaktionsstrecke **(1000)** vorgelegt wird, befindet sich demnach im erfindungsgemäßen Verfahren während der Durchführung von Schritt (A) (I) kein Isocyanat **(1)** in der Reaktionsstrecke **(1000).**

Nachstehend werden verschiedene Ausführungsformen der Erfindung näher beschrieben. Diese können dabei beliebig miteinander kombiniert werden, solange sich für den Fachmann aus dem Kontext nicht eindeutig etwas anderes ergibt.

**Schritt (A)** des erfindungsgemäßen Verfahrens betrifft den Anfahrvorgang der Reaktionsstrecke **(1000)** ausgehend von einer nicht im Betrieb befindlichen Phosgenierungsanlage. In Schritt (A) des erfindungsgemäßen Verfahrens wird nun der jeweils vorliegende Ausgangszustand so in den Zustand der Produktion unter Normalbedingungen überführt, dass die eingangs genannten Probleme nicht oder allenfalls geringfügig auftreten, wie nachfolgend im Detail ausgeführt wird:

In einer **ersten Ausführungsform** des erfindungsgemäßen Verfahrens umfasst die Reaktionsstrecke **(1000)** lediglich eine Mischzone **(1100)** und eine dieser strömungstechnisch nachgeordnete Reaktionszone **(1200),** wobei beide auch in einem einzigen Apparat vereinigt sein können, sowie Peripheriegeräte wie Rohrleitungen, Pumpen, Heizungen und dgl. mehr. Geeignete Apparate für die Mischzone **(1100)** sind dem Fachmann bekannte statische oder dynamische Mischaggregate, wie sie beispielsweise in EP 2 077 150 B1 (Rotor-Stator-Mischer; siehe insbesondere die Zeichnungen und die zugehörigen Textpassagen) und in DE 37 44 001 C1 (Mischdüse, siehe insbesondere die Zeichnungen und die zugehörigen Textpassagen) festgelegt sind. Geeignete Apparate für die Reaktionszone **(1200)** sind dem Fachmann bekannt, bspw. vertikale Rohrreaktoren, die bevorzugt durch horizontale Lochbleche unterteilt sind und optional - bei isothermer Verfahrensführung - beheizt werden können, optional verbunden mit einem nachgeschalteten Abscheider zur Trennung von Gas- und Flüssigphase, wie in EP 0 716 079 B1 mit Einbauten im Reaktor oder in EP 1 601 456 B1 ohne Einbauten im Reaktor, beschrieben.

Misch- und Reaktionszone werden in **Schritt (A)** (**I**) mit inertem Lösungsmittel **(4)** und Phosgen **(3)** zumindest teilweise beschickt. Phosgen **(3)** wird dabei bevorzugt als Phosgenlösung **(30),** d. h. eine Lösung von Phosgen **(3)** im inerten Lösungsmittel **(4),** zugeführt, wie in FIG. 1 dargestellt. Der Massenanteil an Phosgen **(3)** in dieser Phosgenlösung **(30)** beträgt bevorzugt 3,0 % bis 95 %, besonders bevorzugt 20 % bis 75 %. Zur Herstellung der Phosgenlösung **(30)** können geeignete Mischaggregate zur Vermischung von Phosgen **(3)** und inertem Lösungsmittel **(4)** zur Anwendung kommen (in FIG. 1 nicht gezeigt). Geeignete Apparate dafür sind aus dem Stand der Technik bekannt. Geeignete erfindungsgemäße inerte *Lösungsmittel **(4)*** sind dabei unter den Reaktionsbedingungen inerte Lösungsmittel wie z. B. Monochlorbenzol, Dichlorbenzol (insbesondere das *ortho*-Isomer), Dioxan, Toluol, Xylol, Methylenchlorid, Perchlorethylen, Trichlorfluormethan oder Butylacetat. Das inerte Lösungsmittel **(4)** ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Chlorbenzol oder *ortho*-Dichlorbenzol eingesetzt.

In der Variante (i) werden zunächst die Mischzone **(1100)** und die Reaktionszone **(1200)** mit inertem Lösungsmittel **(4)** zumindest teilweise beschickt und danach auf die angestrebte Soll-Temperatur, die bevorzugt einen Wert von 80 °C bis 130 °C, besonders bevorzugt von 95 °C bis 115 °C, hat, aufgeheizt. Die Zugabe des inerten Lösungsmittels **(4)** in diesem Teilschritt kann, wie in FIG. 1 dargestellt, durch die Leitung, die zur Vermischung von Phosgen **(3)** und inertem Lösungsmittel **(4)** führt, erfolgen, wobei dann die Phosgenzufuhr **(3)** währenddessen unterbrochen ist. Es ist aber auch möglich (in FIG. 1 gestrichelt gezeichnet), eine eigene Zuführleitung nur für das inerte Lösungsmittel **(4)** vorzusehen. Anschließend wird das inerte Lösungsmittel **(4)** mit Phosgen **(3)** versetzt, bevorzugt wie in FIG. 1 dargestellt in Form einer Phosgenlösung **(30),** und zwar bevorzugt so lange, bis sich in der Reaktionszone **(1200)** ein Massenanteil an Phosgen **(3)** von 0,5 % bis 20 %, besonders bevorzugt von 1 % bis 10 %, bezogen auf die Gesamtmasse von Phosgen **(3)** und inertem Lösungsmittel **(4),** einstellt. Sobald die angestrebte Soll-Temperatur und der angestrebte Massenanteil an Phosgen **(3)** im inerten Lösungsmittel **(4)** erreicht sind, wird durch Zugabe von Amin **(2),** bevorzugt wie in FIG. 1 gezeigt in Form einer Aminlösung **(20),** d. h. einer Lösung des Amins (2) im inerten Lösungsmittel **(4),** und weiteren Phosgens **(3),** bevorzugt weiterer Phosgenlösung **(30),** die Phosgenierung gestartet. Der Massenanteil des Amins **(2)** in der Aminlösung **(20)** beträgt dabei bevorzugt 5,0 % bis 95 %, besonders bevorzugt 20 % bis 70 %. Es hat sich bewährt, die Reaktionsstrecke **(1000)** vor Durchführung des Schritts (A) mit inertem Lösungsmittel **(4)** zu spülen.

In der Variante (ii) werden inertes Lösungsmittel **(4)** und Phosgen **(3)** in die Mischzone **(1100)** und die Reaktionszone **(1200)** eingeführt, bevor auf die angestrebte Soll-Temperatur, die auch in dieser Ausführungsform bevorzugt einen Wert von 80 °C bis 130 °C, besonders bevorzugt von 95 °C bis 115 °C, hat, aufgeheizt wird. Bevorzugt geschieht dies so, dass zunächst eine Lösung von Phosgen **(3)** im inerten Lösungsmittel **(4)** hergestellt wird (Phosgenlösung **(30)**), wobei der Massenanteil an Phosgen **(3)** im inerten Lösungsmittel **(4)** bevorzugt 0,5 % bis 20 %, besonders bevorzugt von 1 % bis 10 %, bezogen auf die Gesamtmasse von Phosgen und inertem Lösungsmittel, beträgt. Diese Phosgenlösung **(30)** wird über die Mischzone **(1100)** in die Reaktionszone **(1200)** eingeführt, wie in FIG. 1 gezeigt.

In beiden Varianten (i) und (ii) wird bevorzugt so verfahren, dass am Ende von Schritt A (I) mindestens 50 Vol.-%, bevorzugt mindestens 80 Vol.-%, besonders bevorzugt mindestens 99 Vol.-%, ganz besonders bevorzugt 100 Vol.-% des für die Umsetzung des Amins **(2)** mit Phosgen **(3)** im inerten Lösungsmittel **(4)** zur Verfügung stehenden Innenvolumens der Reaktionszone **(1200)** mit der Mischung aus Amin **(2),** Phosgen **(3)** und inertem Lösungsmittel **(4)** beschickt sind. Das "für die Umsetzung des Amins **(2)** mit Phosgen **(3)** im inerten Lösungsmittel **(4)** zur Verfügung stehende Innenvolumen der Reaktionszone **(1200)**" reicht in der Reaktionszone **(1200)** in der Ausgestaltung gemäß FIG. 1 bis zur gepunkteten Linie in Höhe der Leitung für das flüssige Rohprodukt **(61).**

Die Aminzugabe in Schritt A (II) wird erst gestartet, wenn die Misch- und Reaktionszone zumindest teilweise mit inertem Lösungsmittel und Phosgen beschickt sind und die Soll-Temperatur der Umsetzung erreicht ist. Hierdurch wird bewirkt, dass zu Beginn von Schritt A (II) ein sehr hoher molarer Überschusses von Phosgen **(3)** gegenüber dem Amin **(2)** vorliegt, wodurch die Gefahr der Bildung von Filmen und Ablagerungen an den Apparatewänden in der Reaktionsstrecke vermindert wird.

Erfindungsgemäß geeignete Amine **(2),** die durch das beschriebene Verfahren zu den entsprechenden Isocyanaten umgesetzt werden, sind Methylendiphenyldiamin, Polymethylenpolyphenylpolyamin, Gemische aus Methylendiphenyldiamin und Polymethylenpolyphenylpolyamin, Toluylendiamin, Xylylendiamin, Hexamethylendiamin, Isophorondiamin und Naphthyldiamin. Bevorzugt sind Methylendiphenyldiamin, Gemische aus Methylendiphenyldiamin und Polymethylenpolyphenylpolyamin sowie Toluylendiamin.

In **Schritt (A) (II)** ist es bevorzugt, eine Lösung des Amins **(2)** in einem inerten Lösungsmittel **(4)** herzustellen (Aminlösung **(20)**) und diese gemeinsam mit einer Lösung von Phosgen **(3)** in einem inerten Lösungsmittel **(4)** (Phosgenlösung **(30)**) der Mischzone **(1100)** zuzuführen, wie in FIG. 1 gezeigt. Zweckmäßigerweise wird man für das Amin und das Phosgen das gleiche Lösungsmittel **(4)** wählen, obwohl dies nicht unbedingt erforderlich ist. Der Massenanteil des Amins **(2)** in der Aminlösung **(20)** beträgt dabei bevorzugt 5,0 % bis 95 %, besonders bevorzugt 20 % bis 70 %, bezogen auf die Gesamtmasse von Amin **(2)** und inertem Lösungsmittel **(4).** Der Massenanteil des Phosgens **(3)** in der Phosgenlösung **(30)** beträgt dabei bevorzugt 3,0 % bis 95 %, besonders bevorzugt 20 % bis 75 %, bezogen auf die Gesamtmasse von Phosgen **(3)** und inertem Lösungsmittel **(4).**

Die eingesetzten Phosgen- und Aminlösungen werden bevorzugt vor dem Einbringen in Mischzone **(1200)** temperiert, und zwar so, dass die Mischtemperatur vor Einsetzen der Phosgenierungsreaktion bzw. der Reaktion von Amin **(2)** und gebildetem HCl zum entsprechenden Aminhydrochlorid ausreichend hoch ist, um eine Separation der Aminlösung in zwei Phasen zu vermeiden. Eine derartige Phasenseparation führt zu einem lokalen Aminüberschuss, der zu einer erhöhten Bildung von Harnstoffen aus Amin und Phosgen und somit zu einer verstärkten Feststoffbildung bis hin zum Verstopfen des Mischaggregates führen kann. Dieses Phänomen kann in bestimmten Temperaturbereichen beobachtet werden. Bevorzugt weist daher die Phosgenlösung **(30)** eine Temperatur von -20 °C bis +80 °C, besonders bevorzugt von -10 °C bis +20 °C, auf. Ganz besonders bevorzugt liegt die Temperatur der Phosgenlösung **(30)** im Bereich von -5 °C bis +10 °C. Die Aminlösung **(20)** wird bevorzugt auf eine Temperatur von +25 °C bis +160 °C, besonders bevorzugt +40 °C bis +140 °C, temperiert. Ganz besonders bevorzugt liegt die Temperatur der Aminlösung im Bereich von +50 °C bis +120 °C. Bevorzugt erfolgt die Temperierung und Dosierung der Eduktlösungen bei einer Druckstufe, die oberhalb des Dampfdruckes der jeweiligen Lösung liegt. Dabei kann ein absoluter Druck von 1,0 bar bis 70 bar, bevorzugt von 2,0 bar bis 45 bar, ganz besonders bevorzugt von 3 bar bis 25 bar, eingestellt werden.

Konzentrationen und Mengenströme der Edukte Amin und Phosgen werden in Schritt (A) (II) bevorzugt so gewählt, dass sich in der Mischzone **(1100)** nach vollständiger Verdrängung des in Schritt A (I) vorgelegten Gemisches aus Amin **(2),** Phosgen **(3)** und inertem Lösungsmittel **(4)** ein molares Verhältnis von Phosgen zu primären Aminogruppen von 1,1 : 1 bis 30 : 1, besonders bevorzugt von 1,25 : 1 bis 3 : 1, einstellt.

In einer **zweiten Ausführungsform** des erfindungsgemäßen Verfahrens ist zwischen Mischzone **(1100)** und Reaktionszone **(1200)** eine zusätzliche Verweilzeiteinrichtung (**1110;** in FIG. 1 gestrichelt gezeichnet) zur Optimierung der Vermischung der Edukte Amin und Phosgen vorhanden. Diese ist im einfachsten Fall ein Rohr, dessen Durchmesser und Länge der angestrebten Produktionskapazität der Reaktionsstrecke **(1000)** angepasst werden. Die in Schritt (A) (I) zuzugebenden Edukte inertes Lösungsmittel **(4)** und Phosgen **(3)** gelangen über die Mischzone **(1100)** durch die Verweilzeiteinrichtung **(1110)** in die Reaktionszone **(1200),** d. h. auch die Verweilzeiteinrichtung **1110** wird vor Aminzugabe mit inertem Lösungsmittel und Phosgen gemäß Variante (i) oder (ii), bevorzugt vollständig, beschickt, um die Reaktionszone **(1200)** zumindest teilweise mit inertem Lösungsmittel und Phosgen beschicken zu können. Alle für die erste Ausführungsform genannten Vorzugsbereiche (Lösungsmittelreinheit, Druck, Temperatur, Amin- und Phosgenmassenanteil in den jeweiligen Lösungen, molares Verhältnis von Phosgen zu primären Aminogruppen) gelten für diese Ausführungsform gleichermaßen. Gleiches gilt für die als bevorzugt gekennzeichneten Einsatzstoffe und Apparate.

In einer **dritten Ausführungsform** des erfindungsgemäßen Verfahrens, die mit beiden vorgenannten Ausführungsformen kombiniert werden kann, ist der Reaktionszone **(1200)** eine Vorrichtung **(1300)** zur Spaltung des bei Flüssigphasenphosgenierungen von Aminen auftretenden Zwischenprodukts Carbaminsäurechlorid nachgeschaltet (in FIG. 1 gestrichelt gezeichnet). Dies ist dann vorteilhaft, wenn das aus der Reaktionszone **(1200)** austretende, nach Abtrennung der Chlorwasserstoff und Phosgen enthaltenden Gasphase **(71)** erhaltene flüssige Rohprodukt **(61)** noch substanzielle Anteile an nicht gespaltenem Carbaminsäurechlorid enthält. Geeignete Vorrichtungen **1300** sind dem Fachmann bekannt; als Beispiele bei denen der Flüssigkeitsfilm mechanisch erzeugt wird, beispielsweise sogenannte SAMBAY- und LUWA-Dünnschichtverdampfer sowie Sako-Dünnschichtverdampfer und ALFA-LAVAL-Centrithermverdampfer genannt. Es können auch Schichtverdampfer verwendet werden, die keine bewegten Teile aufweisen. Beispiele hierfür sind Fallfilmverdampfer (auch als Fallstromverdampfer oder Fallschichtverdampfer bezeichnet) oder auch Wendelrohrverdampfer und Kletterverdampfer. In der Vorrichtung **1300** wird im flüssigen Rohprodukt **61** noch vorhandenes Carabaminsäurechlorid in das gewünschte Isocyanat und Chlorwasserstoff gespalten. In der Vorrichtung **1300** wird so ein Isocyanat-haltiger flüssiger Strom **(62)** und ein Gasstrom **(72)** enthaltend Chlorwasserstoff (und ggf. überschüssiges Phosgen) erhalten.

In dieser Ausführungsform ist ein Beschicken der Vorrichtung **1300** mit Phosgen **(3)** und inertem Lösungsmittel **(4)** ehe in Schritt (A) (II) die Zugabe des Amins **(2)** gestartet wird, nicht zwingend erforderlich. Alle für die erste Ausführungsform genannten Vorzugsbereiche (Lösungsmittelreinheit, Druck, Temperatur, Amin- und Phosgenmassenanteil in den jeweiligen Lösungen, molares Verhältnis von Phosgen zu primären Aminogruppen) gelten für diese Ausführungsform gleichermaßen. Gleiches gilt für die als bevorzugt gekennzeichneten Einsatzstoffe und Apparate.

Nach Erreichen des angestrebten Betriebszustands erfolgt die kontinuierliche Produktion des Isocyanats **(Schritt (B)**) in der Reaktionsstrecke **(1000).** Schritt (B) kann nach einem aus dem Stand der Technik bekannten Verfahren durchgeführt werden. Geeignete Verfahren sind beispielsweise in EP 1 616 857 A1, EP 1 873 142 A1, EP 0 716 079 B1 oder EP 0 314 985 B1 beschrieben, die grundsätzlich ohne besondere Vorkehrungen auf den Schritt (B) des erfindungsgemäßen Verfahren angewandt werden können. Konzentrationen und Mengenströme der Edukte Amin **(2)** und Phosgen **(3)** werden dabei jedoch bevorzugt so gewählt, dass sich in der Mischzone **(1100)** ein molares Verhältnis von Phosgen zu primären Aminogruppen von 1,1 : 1 bis 30 : 1, besonders bevorzugt von 1,25 : 1 bis 3 : 1, einstellt. Des Weiteren werden bevorzugt auch die für Schritt (A) beschriebenen bevorzugten Ausgestaltungen betreffend Lösungsmittelreinheit, Druck, Temperatur, Amin- und Phosgenmassenanteil in den jeweiligen Lösungen auch in Schritt (B) eingehalten.

Alle Verfahren für die kontinuierliche Produktion eines Isocyanats in der Flüssigphase liefern ein Rohprodukt, das eine Flüssigphase, enthaltend neben dem gewünschten Isoyanat gelösten Chlorwasserstoff und überschüssiges gelöstes Phosgen, sowie eine Gasphase, enthaltend Chlorwasserstoffgas und überschüssiges Phosgen, umfasst. Nach Abtrennung der Gasphase **71** (bspw. bei Austritt aus der Reaktionszone **(1200)** wie in FIG. 1 gezeigt oder in einem geeigneten Abscheider, der sich an die Reaktionszone anschließt) verbleibt eine flüssige Isocyanat-Rohlösung **(61),** die, ggf. nach Durchlaufen einer Vorrichtung zur Spaltung von Carbaminsäurechlorid **(1300),** nach aus dem Stand der Technik bekannten Verfahren weiter aufgearbeitet wird (Abtrennung des Lösungsmittels, Feinreinigung des Isocyanats - in FIG. 1 rein schematisch als Aufarbeitungszone **2000** dargestellt), um das gewünschte Isocyanat **(1)** in möglichst hoher Reinheit zu gewinnen. Geeignete Verfahren sind beschrieben in EP 1 854 783 A2 und EP 1 506 957 A1, oder auch in EP 1 371 635 B1.

Dem Fachmann ist bekannt, dass auch eine grundsätzlich kontinuierliche Produktion nicht beliebig lange betrieben werden kann, sondern in bestimmten Abständen unterbrochen werden muss, bspw. zur Durchführung von Wartungsarbeiten. Das Abfahren einer kontinuierlichen Isocyanat-Produktion in einer Art und Weise, welche die eingangs genannten Probleme bei Wiederinbetriebnahme vermeidet oder zumindest minimiert, ist Gegenstand von **Schritt (C)** des erfindungsgemäßen Verfahrens.

Erfindungsgemäß wesentlich ist nun, dass zum Abfahren der kontinuierlichen Produktion zunächst nur die Zufuhr des Amins **(2)** beendet wird, während Phosgen **(3)** und inertes Lösungsmittel **(4)** für einen Zeitraum t_{C} noch weiter kontinuierlich zugeführt werden. Dadurch, dass weiterhin Phosgen **(3)** und inertes Lösungsmittel **(4),** bevorzugt als Phosgenlösung **(30),** aufgegeben werden, erreicht man einen übergroßen Phosgenüberschuss durch den alle noch in der Reaktionsstrecke **(1000)** vorhandenen Zwischenprodukte wie Aminhydrochlorid und Carbaminsäurechlorid abreagieren. Bevorzugt wird dabei der Zeitraum t_{C} so gewählt, dass das für die Umsetzung des Amins **(2)** mit Phosgen **(3)** im inerten Lösungsmittel **(4)** zur Verfügung stehende Innenvolumen der Reaktionszone **(1200)** 0,1-mal bis 10-mal, bevorzugt 1-mal bis 5-mal, mit Phosgen **(1)** und inertem Lösungsmittel **(4),** bevorzugt in Form der Phosgenlösung **(30),** durchlaufen wird. Bei Einhaltung dieser Werte werden die Mischzone **(1100)** und sofern vorhanden die Verweilzeiteinrichtung **(1110)** in der Regel wesentlich häufiger durchlaufen, da diese im Allgemeinen wesentlich kleiner als die Reaktionszone **(1200)** sind. Je gründlicher dieser Vorgang durchgeführt wird, desto geringer ist die Gefahr der Bildung von Filmen und Ablagerungen. Dabei kann die Reaktionsstrecke **(1000)** ganz oder teilweise mittels technischer Heizung beheizt werden, wobei bevorzugt maximal die Temperaturen aus der kontinuierlichen Fahrweise (Schritt (B)) beibehalten werden. Nach Durchführung des Schrittes (C) befinden sich also nur noch Phosgen **(3)** und inertes Lösungsmittel **(4)** in der Reaktionsstrecke. Isocyanat **(1)** und evtl. vorhandenes nicht umgesetztes Amin **(2)** sowie evtl. vorhandene Zwischenprodukte werden durch den Schritt (C) aus der Reaktionsstrecke herausgespült.

Nachdem der gewünschte Volumenaustausch in Schritt (C) erfolgt ist, wird abschließend bevorzugt **in einem Schritt (D)** das Phosgen **(3)** mit inertem Lösungsmittel **(4)** aus der Reaktionsstrecke **(1000)** verdrängt. Zu diesem Zweck wird zunächst nur die Zufuhr des Phosgens **(3)** beendet, während inertes Lösungsmittel noch weiter kontinuierlich zugeführt wird. Zur Erzielung eines nachhaltigen Effektes sollte die Dauer t_{D} dieser Lösungsmittelwäsche bevorzugt so gewählt werden, dass das für die Umsetzung des Amins **(2)** mit Phosgen **(3)** im inerten Lösungsmittel **(4)** zur Verfügung stehende Innenvolumen der Reaktionszone **(1200)** 0,1-mal bis 10-mal, besonders bevorzugt 1-mal bis 5-mal, mit inertem Lösungsmittel **(4)** durchlaufen wird. Bei Einhaltung dieser Werte werden die Mischzone **(1100)** und sofern vorhanden die Verweilzeiteinrichtung **(1110)** in der Regel wesentlich häufiger durchlaufen, da diese im Allgemeinen wesentlich kleiner als die Reaktionszone **(1200)** sind. Auch Durchspülungsdauern von mehreren Tagen können im Sinne der vorliegenden Erfindung angewandt werden und vorteilhaft sein. Die zu wählende Lösungsmittelmenge und Spüldauer hängt neben dem Apparatevolumen der Reaktionsstrecke **(1000)** inklusive Peripheriegeräten auch, sofern nicht völlig vermeidbar, von der Menge an evtl. vorhandenen Ablagerungen ab.

Durch die erfindungsgemäße Vorgehensweise ergeben sich die folgenden Vorteile für die Herstellung von Isocyanaten:
i) Die Produktivität der Reaktionsstrecke ist höher, weil weniger Reinigungszeiten nötig sind.
ii) Die Produktivität der Reaktionsstrecke ist höher, weil weniger Druckverluste in den Mischaggregaten und Rohrleitungen auftreten.
iii) Die Energieeffizienz der Reaktionsstrecke ist höher, weil weniger Ablagerungen an den Apparatewänden eine bessere Wärmeübertragung gewährleisten.
iv) Es entsteht weniger Abfall nach der Reinigung der Reaktionsstrecke (Polyharnstoffbildung minimiert).
v) Die Bildung von Feststoffen, die die nachgeschalteten Apparate wie Pumpen und Kolonnen durch Abrasion oder Ablagerungen beeinträchtigen können, wird minimiert.

Somit ermöglicht das erfindungsgemäße Verfahren durch Sicherstellung eines übergroßen Überschusses von Phosgen **(3)** gegenüber dem Amin **(2)** zu Beginn des Schrittes A (II) einen technisch reibungslosen Start der Reaktionsstrecke ohne Ausfallzeiten mit direkt hoher Endproduktqualität des angestrebten Isocyanats. Das erfindungsgemäße Verfahren ermöglicht auch ein zügigeres Anfahren und somit eine schnellere Steigerung des Amin-Mengenstroms und dadurch eine erhöhte Produktion.

### Beispiele

### Allgemeine Bedingungen für die Herstellung von einem Gemisch aus Methylendiphenyldiisoyanat und Polymethylenpolyphenylpolisocyanat (nachfolgend summarisch MDI) bei "eingefahrener" Produktionsanlage (entsprechend Schritt (B) des erfindungsgemäßen Verfahrens)

4,3 t/h eines Gemisches aus Methylendiphenyldiamin und Polymethylenpolyphenylpolyamin (nachfolgend summarisch MDA; **2**) einer Temperatur von 110 °C werden mit 11 t/h Monochlorbenzol (MCB; **4**) einer Temperatur von 30 °C als Lösungsmittel über einen statischen Mischer **(1100)** zu einer 28%igen MDA-Lösung **(20)** vermischt. Phosgen **(3)** wird bereitgestellt über einen Phosgengenerator und einen Phosgenverflüssiger. Im Anschluss wird das Phosgen **(3)** in einem Phosgenlösungstank mit MCB **(4)** auf eine 35%ige Phosgenlösung **(30)** eingestellt. Stündlich werden 24 Tonnen 35%ige Phosgenlösung **(30)** einer Temperatur von 0 °C mit 4,3 Tonnen MDA **(2)** in Form der 28%igen MDA-Lösung **(20)** einer Temperatur von 45 °C in einer adiabaten Reaktion, wie in EP 1 873 142 B1 beschrieben, umgesetzt. Nach der Durchmischung der beiden Rohstofflösungen im Mischaggregat **(1100)** wird die erhaltene Reaktionslösung **(5)** mit einer Temperatur von 85 °C über eine Suspensionsleitung **(1200)** in einen beheizten Phosgenierturm **(1200)** gefahren. Am Kopf des Phosgenierturmes liegen ein absoluter Druck von 1,6 bar und eine Temperatur von 111 °C vor. Der bei der Reaktion entstandene Chlorwasserstoff wird zusammen mit Spuren an Phosgen und MCB als Gasstrom **(71)** abgeführt. Das flüssige Reaktionsgemisch **(61)** wird dem Phosgenierturm **(1200)** entnommen und der Aufarbeitungssequenz **(2000)** zugeführt. Dazu wird es zunächst als Seitenstrom in eine beheizte Entphosgenierkolonne eingeleitet. Bei einer Kopftemperatur von 116 °C und einem absoluten Druck von 1,6 bar wird über Kopf Phosgen mit Spuren MCB und Chlorwasserstoff abgeführt. Phosgen wird in einer Phosgenabsorptionskolonne absorbiert und in den Phosgenlösungstank gefahren, und Chlorwasserstoff wird in einen Chlorwasserstoffabsorber und dann zur weiteren Verwendung in einen Salzsäuretank geleitet. Nach Abtrennung von Chlorwasserstoff und überschüssigem Phosgen aus der Isocyanat enthaltenden Reaktionslösung wird eine Isocyanat-Rohlösung erhalten, die aus dem Sumpf der Entphosgenierkolonne ausgetragen und mit einer Temperatur von 155 °C in eine erste Destillationsstufe gefahren wird, um sie vom Lösungsmittel MCB zu befreien. Der absolute Druck am Kopf dieser Lösungsmitteldestillationskolonne beträgt 800 mbar bei einer Sumpftemperatur von 155 °C. Über Kopf wird MCB gasförmig abgezogen, wobei dieser MCB-Gasstrom mit kaltem MCB (30 °C) in einer Waschkolonne besprüht wird, um einen möglichen Mitriss von Isocyanat in die Vakuumleitungen zu verhindern. Das Reaktionsprodukt wird aus dem Sumpf der Kolonne ausgetragen und in einer zweiten Kolonne bis auf 1 % von restlichem MCB befreit. Anschließend wird in einem Gegenstromverdampfer bei einem absoluten Druck von 20 mbar und einer Sumpftemperatur von 210 °C das Produkt von Nebenkomponenten wie Phenylisocyanat und restlichem MCB befreit. Dabei fallen als Sumpfprodukt 5,4 t/h MDI an, das mittels weiterer Destillation zu MDI der gewünschten Reinheit **(1)** aufgearbeitet und anschließend zur weiteren Verwendung in einen Tank gefahren wird.
So hergestelltes MDI hat einen Restlösemittelgehalt an MCB von < 5 ppm (GC), einen Gehalt an hydrolisierbarem Chlor von < 100 ppm (nach Solvolyse mittels Titration) und einen Gehalt von gebundenem Chlor von < 50 ppm (Wickboldt-Verbrennung).

### Beispiel 1 (Vergleichsbeispiel, Schritt (C) nicht erfindungsgemäß)

Die Herstellung von 5,4 t/h MDI in kontinuierlicher Fahrweise wurde, wie in den allgemeinen Bedingungen beschrieben, bei Nennlast durchgeführt. Die Anlage wurde abgefahren, wobei die Phosgenlösungs- und die MDA-Lösungszufuhr *gleichzeitig* abrupt abgestellt wurden. Man ließ den Reaktor abkühlen, wobei der Reaktordruck mit Stickstoff konstant gehalten wurde. Nach eintägigen Reparaturarbeiten an einem anderen Anlagenteil wurde die Phosgenieranlage zum Anfahren der Anlage mit Lösungsmittel bis zur Höhe der Entnahmeleitung für das Rohprodukt **(61)** befüllt und mit Hilfe eines Wärmeträgers auf 105 °C aufgeheizt. Die Phosgenlösungszufuhr wurde mit einer Last von 25% der Nennlast in Betrieb genommen. Nach einer Stunde wurde die MDA-Lösungszufuhr mit einer Last von 15 % der Nennlast gestartet, was einer Produktionsleistung von 0,8 t/h (MDI) entsprach. Die beiden Mengenströme sollten dann innerhalb von zwei Stunden auf Nennlast erhöht werden. Dies gelang nicht aufgrund von Feststoffanbackungen, die sich nach dem abrupten Abstellen im Bereich des Phosgenierreaktors und am Mischaggregat gebildet hatten. Der zur Verfügung stehende Vordruck der Edukte **20** und **30** reichte nicht mehr aus, um die gewünschte Nennlast zu erreichen. Die Anlage musste abgefahren und die mit Feststoffen belegten Bereiche gereinigt werden.

### Beispiel 2 (Vergleichsbeispiel, Schritt (A) nicht erfindungsgemäß)

Die Herstellung von 5,4 t/h MDI in kontinuierlicher Fahrweise wurde, wie in den allgemeinen Bedingungen beschrieben, bei Nennlast durchgeführt. Die Anlage wurde abgefahren, indem zuerst die MDA-Zufuhr abgestellt wurde. MCB aus der MDA-Lösungszufuhr und die Phosgenlösung liefen für eine Stunde mit der vorab laufenden Nennlastmenge weiter. Anschließend wurde die Phosgenzufuhr abgestellt, und mit zweistündigem Spülen mit MCB wurde die Anlage von Phosgen befreit. Die Temperatur der Phosgenieranlage wurde mittels technischer Heizung bei 110 °C gehalten. Nun ließ man die Phosgenieranlage abkühlen, wobei der Anlagendruck mit Stickstoff konstant gehalten wurde. Nach mehrtägigen Reparaturarbeiten an einem anderen Anlagenteil wurde die Phosgenieranlage zum Anfahren der Anlage mit Lösungsmittel bis zur Höhe der Entnahmeleitung für das Rohprodukt **(61)** befüllt und mit Hilfe eines Wärmeträgers auf 105 °C aufgeheizt. Die Phosgenlösungs- und MDA-Lösungszufuhr wurden *gleichzeitig* angestellt. Die Anlage wurde mit 15 % der Nennkapazität gestartet, was einer Produktionsleistung von 0,8 t/h (MDI) entsprach. Die Mengenströme wurden dann innerhalb von zwei Stunden auf Nennlast erhöht, und die Anlage wurde in den kontinuierlichen Modus (Schritt (B)) überführt. Nach weiteren fünf Stunden bei Nennlast musste die Phosgenieranlage komplett abgestellt werden, weil die Verteilerböden der Entphosgenierkolonne zu verstopften begannen und dadurch der Druckverlust über die Kolonne anstieg. Die Anlage musste abgefahren werden, um die Entphosgenierkolonne von angebackenem und lose in der Kolonne liegendem Harnstoff zu befreien und für ein neuerliches Anfahren vorzubereiten.

### Beispiel 3 (erfindungsgemäß)

Die Herstellung von 5,4 t/h MDI in kontinuierlicher Fahrweise wurde, wie in den allgemeinen Bedingungen beschrieben, bei Nennlast durchgeführt. Die Anlage wurde abgefahren, indem zuerst die MDA-Zufuhr abgestellt wurde. MCB aus der MDA-Lösungszufuhr und die Phosgenlösung liefen für eine Stunde mit der vorab laufenden Nennlastmenge weiter. Anschließend wurde die Phosgenzufuhr abgestellt, und mit zweistündigem Spülen mit Lösungsmittel wurde die Anlage von Phosgen befreit. Die Temperatur der Phosgenieranlage wurde mittels technischer Heizung bei 110 °C gehalten. Nun ließ man die Phosgenieranlage abkühlen, wobei der Anlagendruck mit Stickstoff konstant gehalten wurde. Nach mehrtägigen Reparaturarbeiten an einem anderen Anlagenteil wurde die Phosgenieranlage zum Anfahren der Anlage mit Lösungsmittel bis zur Höhe der Entnahmeleitung für das Rohprodukt **(61)** befüllt und mit Hilfe eines Wärmeträgers auf 105 °C aufgeheizt. Die Phosgenlösungszufuhr wurde mit einer Last von 25 % der Nennlast in Betrieb genommen. Nach einer Stunde wurde die MDA-Lösungszufuhr mit einer Last von 15 % der Nennlast gestartet, was einer Produktionsleistung von 0,8 t/h (MDI) entsprach. Die beiden Mengenströme wurden dann innerhalb von zwei Stunden auf Nennlast erhöht, und anschließend wurde die Phosgenieranlage, wie in den allgemeinen Bedingungen beschrieben, für mehrere Monate betrieben. Das Anfahren gelang unmittelbar mit spezifikationsgerechter Ware.

Wie die Beispiele zeigen, entstehen bei bereits vorhandenen Anbackungen im Phosgenierreaktor während des Anfahrens der Phosgenierung große Probleme mit der Eduktzufuhr in die Anlage. Bei erfindungsgemäßer Vorgehensweise beim Ab- und Anfahren der Phosgenierung wird demgegenüber die Bildung von Anbackungen und Niederschlägen deutlich reduziert, die Anlage kann über einen langen Produktionszyklus betrieben werden, und es entsteht während der ganzen Zeit spezifikationsgerechte Ware.

## Patentansprüche

1. Kontinuierliches Verfahren zur Herstellung eines Isocyanats (1) durch Umsetzung des korrespondierenden Amins **(2)** mit Phosgen **(3)** in einem inerten Lösungsmittel **(4)** in einer Reaktionsstrecke **(1000),** welche
(a) eine Mischzone **(1100)** zur Vermischung von Amin **(2),** Phosgen **(3)** und inertem Lösungsmittel **(4)**
und
(b) eine strömungstechnisch nach der Mischzone **(1100)** angeordnete Reaktionszone **(1200),**
umfasst;
bei einer Soll-Temperatur T_{Soll}, wobei nacheinander die Schritte
(A) Anfahren der kontinuierlichen Produktion,
(B) kontinuierliche Produktion und
(C) Abfahren der kontinuierlichen Produktion
durchlaufen werden, und wobei
in Schritt (A)
(I) die Mischzone **(1100)** und die Reaktionszone **(1200)** zunächst
(i) nur mit inertem Lösungsmittel **(4)** zumindest teilweise beschickt, dann auf T_{Soll} aufgeheizt und danach zusätzlich mit Phosgen **(3)** aber nicht mit Amin **(2),** bevorzugt zusammen mit weiterem inerten Lösungsmittel **(4),** beschickt werden;
oder
(ii) mit inertem Lösungsmittel **(4)** und Phosgen **(3)** ohne das Amin **(2)** zumindest teilweise beschickt und danach auf T_{Soll} aufgeheizt werden;
(II) erst im Anschluss an Schritt (A) (I) der Reaktionszone **(1200)** das Amin **(2)** sowie weiteres Phosgen **(3)** und weiteres inertes Lösungsmittel **(4)** über die Mischzone **(1100)** kontinuierlich zugeführt werden;
und in Schritt (C) zum Abfahren der kontinuierlichen Produktion zunächst nur die Zufuhr des Amins **(2)** beendet wird, während Phosgen **(3)** und inertes Lösungsmittel **(4)** noch weiter kontinuierlich zugeführt werden.

2. Verfahren nach Anspruch 1, bei dem zwischen Mischzone **(1100)** und Reaktionszone **(1200)** eine zusätzliche Verweilzeiteinrichtung **(1110)** vorhanden ist.

3. Verfahren nach Anspruch 1 oder 2, bei dem der Reaktionszone **(1200)** eine Vorrichtung **(1300)** zur Spaltung von Carbaminsäurechlorid nachgeschaltet ist.

4. Verfahren nach einem der Ansprüche 1 bis 3, bei dem im Anschluss an Schritt (C) der Schritt (D) Verdrängung des Phosgens (3) aus der Reaktionsstrecke **(1000)** durchlaufen wird, welcher durchgeführt wird, indem zunächst nur die Zufuhr des Phosgens **(3)** beendet wird, während inertes Lösungsmittel noch weiter kontinuierlich zugeführt wird.

5. Verfahren nach einem der Ansprüche 1 bis 4, bei dem T_{Soll} 80 °C bis 130 °C, besonders bevorzugt von 95 °C bis 115 °C, beträgt.

6. Verfahren nach einem der Ansprüche 1 bis 5, bei dem in Schritt (A) (I) ein Massenanteil an Phosgen **(3)** von 0,5 % bis 20 %, besonders bevorzugt von 1 % bis 10 %, bezogen auf die Gesamtmasse von Phosgen **(3)** und inertem Lösungsmittel **(4),** eingestellt wird.

7. Verfahren nach einem der Ansprüche 1 bis 6, bei dem Schritt A (I) so durchgeführt wird, dass am Ende dieses Schritts mindestens 50 Vol.-%, bevorzugt mindestens 80 Vol.-%, besonders bevorzugt mindestens 99 Vol.-%, ganz besonders bevorzugt 100 Vol.-% des für die Umsetzung des Amins **(2)** mit Phosgen **(3)** im inerten Lösungsmittel **(4)** zur Verfügung stehenden Innenvolumens der Reaktionszone **(1200)** mit der Mischung aus Amin **(2),** Phosgen **(3)** und inertem Lösungsmittel **(4)** beschickt sind.

8. Verfahren nach einem der Ansprüche 1 bis 7, bei dem in Schritt (C) Phosgen **(1)** und inertes Lösungsmittel **(4)** noch für einen Zeitraum t_{C} weiter kontinuierlich zugeführt werden, wobei der Zeitraum t_{C} so gewählt wird, dass das für die Umsetzung des Amins **(2)** mit Phosgen **(3)** im inerten Lösungsmittel **(4)** zur Verfügung stehende Innenvolumen der Reaktionszone **(1200)** 0,1-mal bis 10-mal, bevorzugt 1-mal bis 5-mal, mit Phosgen **(1)** und inertem Lösungsmittel **(4),** bevorzugt in Form einer Lösung **(30)** von Phosgen **(3)** im inerten Lösungsmittel **(4),** durchlaufen wird.

9. Verfahren nach einem der Ansprüche 4 bis 8, bei dem in Schritt (D) nach Beendigung der Zufuhr des Phosgens **(3)** inertes Lösungsmittel **(4)** noch für einen Zeitraum t_{D} kontinuierlich zugeführt wird, wobei der Zeitraum t_{D} so gewählt wird, dass das für die Umsetzung des Amins **(2)** mit Phosgen **(3)** im inerten Lösungsmittel **(4)** zur Verfügung stehende Innenvolumen der Reaktionszone **(1200)** 0,1-mal bis 10-mal, besonders bevorzugt 1-mal bis 5-mal, mit inertem Lösungsmittel **(4)** durchlaufen wird.

10. Verfahren nach einem der Ansprüche 1 bis 9, bei dem die Konzentrationen und Mengenströme der Edukte Amin und Phosgen in Schritt (A) (II) so gewählt werden, dass sich in der Mischzone **(1100)** nach vollständiger Verdrängung des in Schritt A (I) vorgelegten Gemisches aus Amin **(2),** Phosgen **(3)** und inertem Lösungsmittel **(4)** ein molares Verhältnis von Phosgen zu primären Aminogruppen von 1,1 : 1 bis 30 : 1, besonders bevorzugt von 1,25 : 1 bis 3 : 1, einstellt.

11. Verfahren nach einem der Ansprüche 1 bis 10, bei dem das Amin **(2)** ausgewählt ist aus der Gruppe bestehend aus Methylendiphenyldiamin, Polymethylenpolyphenylpolyamin, einem Gemisch aus Methylendiphenyldiamin und Polymethylenpolyphenylpolyamin, Toluylendiamin, Xylylendiamin, Hexamethylendiamin, Isophorondiamin und Naphthyldiamin.

12. Verfahren nach Anspruch 11, bei dem das Amin **(2)** Methylendiphenyldiamin oder ein Gemisch aus Methylendiphenyldiamin und Polymethylenpolyphenylpolyamin oder Toluylendiamin ist.

13. Verfahren nach Anspruch 11, bei dem das Amin **(2)** Methylendiphenyldiamin oder ein Gemisch aus Methylendiphenyldiamin und Polymethylenpolyphenylpolyamin ist.

14. Verfahren nach Anspruch 11, bei dem das Amin **(2)** Toluylendiamin ist.

15. Verfahren nach einem der Ansprüche 1 bis 14, bei dem das inerte Lösungsmittel **(4)** ausgewählt ist aus der Gruppe bestehend aus Monochlorbenzol, Dichlorbenzol, Dioxan, Toluol, Xylol, Methylenchlorid, Perchlorethylen, Trichlorfluormethan und Butylacetat.

## Claims

1. Continuous process for preparing an isocyanate **(1)** by reacting the corresponding amine **(2)** with phosgene **(3)** in an inert solvent **(4)** in a reaction section **(1000)** comprising
(a) a mixing zone **(1100)** for mixing amine **(2),** phosgene **(3)** and inert solvent **(4)**
and
(b) a reaction zone **(1200)** arranged downstream of the mixing zone **(1100);**
at a target temperature T_{target}, wherein the steps of
(A) starting up continuous production,
(B) continuous production and
(C) shutting down continuous production are run successively, and wherein,
in step (A),
(I) the mixing zone (1100) and the reaction zone **(1200)** are at first at least partly charged
(i) with inert solvent **(4)** only, then heated up to T_{target} and then additionally charged with phosgene **(3)** but not with amine **(2),** preferably together with further inert solvent **(4);**
or
(ii) with inert solvent **(4)** and phosgene **(3)** without the amine **(2)** and then heated up to T_{target};
(II) only after step (A) (I) is the reaction zone **(1200)** supplied continuously with the amine **(2)** and also further phosgene **(3)** and further inert solvent **(4)** via the mixing zone **(1100);**
and, in step (C), the continuous production is shut down by first ending the supply of the amine **(2)** only, while continuous supply of phosgene **(3)** and inert solvent **(4)** still continues.

2. Process according to Claim 1, in which an additional delay device **(1110)** is present between the mixing zone **(1100)** and reaction zone **(1200).**

3. Process according to Claim 1 or 2, in which the reaction zone **(1200)** has a downstream apparatus **(1300)** for cleaving carbamoyl chloride.

4. Process according to any of Claims 1 to 3, in which, after step (C),
step (D) displacing the phosgene (3) from the reaction section **(1000)**
is run, which is conducted by at first ending the supply of phosgene **(3)** only, while continuous supply of inert solvent still continues.

5. Process according to any of Claims 1 to 4, in which Ttarget is 80°C to 130°C, more preferably from 95°C to 115°C.

6. Process according to any of Claims 1 to 5, in which, in step (A) (I), a proportion by mass of phosgene **(3)** of 0.5% to 20%, more preferably of 1% to 10%, based on the total mass of phosgene **(3)** and inert solvent **(4),** is established.

7. Process according to any of Claims 1 to 6, in which step A (I) is conducted in such a way that, at the end of this step, at least 50% by volume, preferably at least 80% by volume, more preferably at least 99% by volume and most preferably 100% by volume of the internal volume of the reaction zone **(1200)** available for the reaction of the amine **(2)** with phosgene **(3)** in the inert solvent **(4)** is charged with the mixture of amine **(2),** phosgene **(3)** and inert solvent **(4).**

8. Process according to any of Claims 1 to 7, in which, in step (C), the supply of phosgene **(1)** and inert solvent **(4)** is still continued for a period of time t_{C}, the period of time tc being chosen such that the internal volume of the reaction zone **(1200)** available for the reaction of the amine **(2)** with phosgene **(3)** in the inert solvent **(4)** is run through 0.1 time to 10 times, preferably 1 time to 5 times, by phosgene **(1)** and inert solvent **(4),** preferably in the form of a solution **(30)** of phosgene **(3)** in the inert solvent **(4).**

9. Process according to any of Claims 4 to 8, in which, in step (D), after the supply of phosgene **(3)** has ended, inert solvent **(4)** is still supplied continuously for a period of time t_{D}, the period of time t_{D} being chosen such that the internal volume of the reaction zone **(1200)** available for the reaction of the amine **(2)** with phosgene **(3)** in the inert solvent **(4)** is run through 0.1 time to 10 times, more preferably 1 time to 5 times, by inert solvent **(4)**.

10. Process according to any of Claims 1 to 9, in which the concentrations and mass flow rates of the amine and phosgene reactants in step (A) (II) are chosen such that, in the mixing zone **(1100),** after complete displacement of the mixture of amine **(2),** phosgene **(3)** and inert solvent **(4)** initially charged in step A (I), a molar ratio of phosgene to primary amino groups of 1.1:1 to 30:1, more preferably of 1.25:1 to 3:1, is established.

11. Process according to any of Claims 1 to 10, in which the amine **(2)** is selected from the group consisting of methylenediphenyldiamine, polymethylenepolyphenylpolyamine, a mixture of methylenediphenyldiamine and polymethylenepolyphenylpolyamine, tolylenediamine, xylylenediamine, hexamethylenediamine, isophoronediamine and naphthyldiamine.

12. Process according to Claim 11, in which the amine **(2)** is methylenediphenyldiamine or a mixture of methylenediphenyldiamine and polymethylenepolyphenylpolyamine or tolylenediamine.

13. Process according to Claim 11, in which the amine **(2)** is methylenediphenyldiamine or a mixture of methylenediphenyldiamine and polymethylenepolyphenylpolyamine.

14. Process according to Claim 11, in which the amine **(2)** is tolylenediamine.

15. Process according to any of Claims 1 to 14, in which the inert solvent **(4)** is selected from the group consisting of monochlorobenzene, dichlorobenzene, dioxane, toluene, xylene, methylene chloride, perchloroethylene, trichlorofluoromethane and butyl acetate.

## Revendications

1. Procédé continu de fabrication d'un isocyanate (1) par mise en réaction de l'amine correspondante (2) avec du phosgène (3) dans un solvant inerte (4) dans une section de réaction (1000), qui comprend
(a) une zone de mélange (1100) pour le mélange de l'amine (2), du phosgène (3) et du solvant inerte (4), et
(b) une zone de réaction (1200) agencée après la zone de mélange (1100) en termes d'écoulement ;
à une température préréglée T_{Soll}, les étapes
(A) le démarrage de la production continue,
(B) la production continue et
(C) la fin de la production continue
étant traversées successivement, et
à l'étape (A),
(I) la zone de mélange (1100) et la zone de réaction (1200) étant tout d'abord
(i) alimentées au moins partiellement avec uniquement le solvant inerte (4), puis portées à T_{Soll}, puis également alimentées avec le phosgène (3), mais pas avec l'amine (2), de préférence conjointement avec du solvant inerte (4) supplémentaire ;
ou
(ii) alimentées au moins partiellement avec le solvant inerte (4) et le phosgène (3) sans l'amine (2), puis portées à T_{Soll} ;
(II) uniquement après l'étape (A) (I), l'amine (2), ainsi que davantage de phosgène (3) et davantage de solvant inerte (4) sont introduits en continu dans la zone de réaction (1200) par le biais de la zone de mélange (1100) ;
et, à l'étape (C), pour la fin de la production continue, tout d'abord uniquement l'introduction de l'amine (2) est terminée, tandis que le phosgène (3) et le solvant inerte (4) sont encore introduits en continu.

2. Procédé selon la revendication 1, selon lequel un dispositif à temps de séjour supplémentaire (1110) est présent entre la zone de mélange (1100) et la zone de réaction (1200).

3. Procédé selon la revendication 1 ou 2, selon lequel un dispositif (1300) pour le clivage de chlorure d'acide carbamique est connecté en aval de la zone de réaction (1200).

4. Procédé selon l'une quelconque des revendications 1 à 3, selon lequel, après l'étape (C), l'étape (D), le refoulement du phosgène (3) de la section de réaction (1000), est traversée, qui est réalisée en ne terminant tout d'abord que l'introduction du phosphène (3), tandis que le solvant inerte est encore introduit en continu.

5. Procédé selon l'une quelconque des revendications 1 à 4, selon lequel T_{Soll} est de 80 °C à 130 °C, de manière particulièrement préférée de 95 °C à 115 °C.

6. Procédé selon l'une quelconque des revendications 1 à 5, selon lequel, à l'étape (A) (I), une proportion en masse de phosgène (3) de 0,5 % à 20 %, de manière particulièrement préférée de 1 % à 10 %, par rapport à la masse totale de phosgène (3) et de solvant inerte (4), est ajustée.

7. Procédé selon l'une quelconque des revendications 1 à 6, selon lequel l'étape A (I) est réalisée de sorte qu'à la fin de cette étape, au moins 50 % en volume, de préférence au moins 80 % en volume, de manière particulièrement préférée au moins 99 % en volume, de manière tout particulièrement préférée 100 % en volume, du volume intérieur de la zone de réaction (1200) disponible pour la réaction de l'amine (2) avec le phosgène (3) dans le solvant inerte (4) est alimenté avec le mélange de l'amine (2), du phosgène (3) et du solvant inerte (4).

8. Procédé selon l'une quelconque des revendications 1 à 7, selon lequel, à l'étape (C), le phosgène (1) et le solvant inerte (4) sont encore introduits en continu pendant une durée t_{C}, la durée t_{C} étant choisie de sorte que le volume intérieur de la zone de réaction (1200) disponible pour la réaction de l'amine (2) avec le phosgène (3) dans le solvant inerte (4) soit traversé 0,1 fois à 10 fois, de préférence 1 fois à 5 fois, avec le phosgène (1) et le solvant inerte (4), de préférence sous la forme d'une solution (30) du phosgène (3) dans le solvant inerte (4).

9. Procédé selon l'une quelconque des revendications 4 à 8, selon lequel, à l'étape (D), après la fin de l'introduction du phosgène (3), le solvant inerte (4) est encore introduit en continu pendant une durée t_{D}, la durée t_{D} étant choisie de sorte que le volume intérieur de la zone de réaction (1200) disponible pour la réaction de l'amine (2) avec le phosgène (3) dans le solvant inerte (4) soit traversé 0,1 fois à 10 fois, de manière particulièrement préférée 1 fois à 5 fois, avec le solvant inerte (4).

10. Procédé selon l'une quelconque des revendications 1 à 9, selon lequel les concentrations et les débits des réactifs amine et phosgène à l'étape (A) (II) sont choisis de sorte qu'un rapport molaire entre le phosgène et les groupes amino primaire de 1,1:1 à 30:1, de manière particulièrement préférée de 1,25:1 à 3:1, s'ajuste dans la zone de mélange (1100) après le refoulement complet du mélange de l'amine (2), du phosgène (3) et du solvant inerte (4) chargé à l'étape A (I).

11. Procédé selon l'une quelconque des revendications 1 à 10, selon lequel l'amine (2) est choisie dans le groupe constitué par la méthylène-diphényldiamine, la polyméthylène-polyphénylpolyamine, un mélange de méthylène-diphényldiamine et de polyméthylène-polyphénylpolyamine, la toluylène-diamine, la xylylène-diamine, l'hexaméthylène-diamine, l'isophorone-diamine et la naphtyldiamine.

12. Procédé selon la revendication 11, selon lequel l'amine (2) est la méthylène-diphényldiamine ou un mélange de méthylène-diphényldiamine et de polyméthylène-polyphénylpolyamine ou la toluylène-diamine.

13. Procédé selon la revendication 11, selon lequel l'amine (2) est la méthylène-diphényldiamine ou un mélange de méthylène-diphényldiamine et de polyméthylène-polyphénylpolyamine.

14. Procédé selon la revendication 11, selon lequel l'amine (2) est la toluylène-diamine.

15. Procédé selon l'une quelconque des revendications 1 à 14, selon lequel le solvant inerte (4) est choisi dans le groupe constitué par le monochlorobenzène, le dichlorobenzène, le dioxane, le toluyène, le xylène, le chlorure de méthylène, le perchloroéthylène, le trichlorofluoroéthane et l'acétate de butyle.
